# EUROPEAN PATENT SPECIFICATION

(11) **EP 3 359 627 B1**
(45) Date of publication and mention of the grant of the patent: **19.08.2020**
(21) Application number: 16781172.8
(22) Date of filing: 06.10.2016
(51) Int. Cl.: C10G 2/00, C07C 29/151

(54) **SUSTAINABLE ENERGY SYSTEM**
NACHHALTIGES ENERGIESYSTEM
SYSTÈME D'ÉNERGIE DURABLE

(30) Priority: 06.10.2015 GB 201517608
(43) Date of publication of application: 15.08.2018
(73) Proprietor: Heptonstall, William B., Edinburgh EH10 5RJ (GB)
(72) Inventor: Heptonstall, William B., Edinburgh EH10 5RJ (GB)
(74) Representative: Chapman, Paul Gilmour
(86) International application number: PCT/GB2016/053107
(87) International publication number: WO 2017/060704

(56) References cited:
- EP-A1- 2 360 230
- AU-A4- 2013 101 029
- US-A1- 2009 165 459
- US-A1- 2013 214 542

## Description

### Field of the Invention

The invention relates to the provision of biomass consuming or burning electric power generating systems that can produce fuels such as methane and methanol for convenient use.

### Background to the Invention

The increasing demand for electricity produced from sustainable or renewable sources of energy presents new challenges in terms of providing the capacity to maintain electricity supply under all load conditions.

Some energy sources, such as solar and wind, are variable and not reliably available. This leads to the requirement for energy storage and/or back up sources of energy.

There is therefore the need for flexible electricity generating and power storage arrangements that can be integrated with the output from variable energy sources to provide confidence in supply.

Biomass provides an opportunity to generate electricity in a renewable fashion. Biomass such as wood can provide the fuel source for electrical power generation with the prospect of secure supply of the fuel by growing more biomass and keeping stocks in hand for use in periods where other energy sources are not sufficient.

Such a use of biomass is described in US2009165459, which discloses a method of producing electrical power for delivery on an electrical power supply grid and includes (a) providing a combined heat and power plant generating heat and electrical power from one or more of biomass, fossil fuel, and nuclear fuel; (b) providing a fuel producing plant for generating a carbon-based fuel and/or a nitrogen-based fuel using electrical power from the combined heat and power plant and a combination of two or more materials selected from the group consisting of carbon dioxide, hydrogen, carbonaceous gasses, carbonaceous liquids, and nitrogen; (c) determining the need for electrical power on the electrical power supply grid and delivering needed electrical power on the grid; and (d) delivering any excess electrical power to the fuel producing plant.

### Description of the Invention

According to a first aspect the present invention provides an energy system comprising:
a biomass fuelled electrical power generating plant;
a carbon capture system for capturing carbon containing flue gases from consuming the biomass as fuel; and
a plant for producing carbon based fuel by reaction of the captured carbon containing flue gases and a source of hydrogen;
   wherein the electrical power generating plant comprises:
   a) a biomass pyrolising plant for pyrolising biomass to a charcoal; and
   b) a direct carbon fuel cell plant for generating electricity from the charcoal.

The biomass fuelled electrical power generation plant may be a plant that burns the biomass, typically with the heat generated used to produce steam in a boiler or boilers for powering electricity generating equipment. Waste heat may be recovered and used as required in the systems described hereafter as required or for local heating systems as known in conventional combined heat and power arrangements.

Typically the biomass will be dried and then pyrolised in a plant that can make use of waste heat from processes described herein that are exothermic. The resulting charcoal product will then be ground to a suitable particle size for consuming in direct carbon fuel cells. In a direct carbon fuel cell the charcoal is oxidised at the anode to give carbon dioxide whilst oxygen is reduced at the cathode. The oxygen may conveniently be supplied in the form of air. The fuel cell produces electricity by the consumption of the charcoal to the flue gas (carbon dioxide).

The carbon based fuel may be methane or methanol. The plant for producing carbon based fuel may be constructed and configured to produce either methane or methanol or both methane and methanol.

Where a biomass burning electrical power generating plant is employed it may be a conventional plant, burning biomass in air in the furnace of a boiler system and generating steam to drive a turbine or turbines that in turn drive an electricity generator or generators. Conveniently the biomass employed is wood. However, as flue gases are captured for conversion to carbon based fuel such as methane or methanol it is convenient to use an oxy-fuel combustion process to burn the biomass in an enriched oxygen atmosphere, in the known fashion. Using pure oxygen and wood as the biomass the flue gas comprises mostly water and carbon dioxide, if combustion is complete or essentially complete. This facilitates the capture of the carbon containing gases, when compared to the use of air, and results in flue gases that are easier to capture and, if required, purify. High temperatures in the boiler furnace that would be generated by use of pure oxygen, or high oxygen containing input gases such as oxygen enriched air, may be conveniently mitigated by recycling flue gases. For example by recycling CO₂, the flue gas mixture as originally produced (or as scrubbed of one or more components). An alternative is to use a staged combustion procedure.

Conveniently a biomass burning electrical power generating plant can operate with air for burning the biomass and optionally run an oxy-fuel combustion process for burning the biomass i.e. the combustion process is switchable from conventional combustion to oxy-fuel.

In addition to producing largely carbon dioxide and water in flue gas, the biomass combustion in the boiler may be adjustable to allow production of carbon monoxide in the flue gas, which can find use as described hereafter. On the other hand, if a proportion of carbon monoxide is present in the flue gas and is not wanted, it can readily be converted to carbon dioxide by a post furnace treatment, such as reaction with water via the water gas shift reaction.

CO + H₂O → CO₂ + H₂

The capture of the carbon containing flue gases may be done in any of the known ways including absorption by amines or by solid sorbents in a suitable plant. Conveniently the carbon capture may be by compression to the liquid phase for carbon dioxide or carbon monoxide in a suitable plant. As the flue gases are cooled for carbon capture, the heat obtained from the cooling process, from a heat exchange system, may be employed for purposes such as drying the biomass before it is put into the furnace of the boiler system, or into the pyrolysis plant, where a charcoal is produced for consumption in direct carbon fuel cells.

The production of methane from the captured carbon is conveniently carried out by means of the Sabatier process or variations thereof. The Sabatier process can be summarised by the equation:

CO₂ + 4H₂ → CH₄ + 2H₂O

The reaction may be carried out at elevated temperature and pressure in the presence of a catalyst such as a nickel catalyst or a ruthenium on alumina catalyst, in the known manner. As the reaction is exothermic large amounts of energy are not required to run the process, provided sources of the input gases are available. As an alternative, if the combustion process produces carbon monoxide, then reaction with hydrogen can be used to produce methane.

For the oxy-fuel combustion process and the production of methane sources of oxygen or at least air enriched in oxygen content and of hydrogen are required. These may be obtained from any source. For example oxygen or air enriched in oxygen may be obtained by use of an air separation plant, in the known manner. Conveniently the oxygen and hydrogen, or at least a proportion of the oxygen and hydrogen required, may be obtained by electrolysis of water. Thus the system may include an electrolysis plant, including appropriate storage for oxygen and hydrogen. An excess of oxygen is likely to be available if electrolysis is used to produce the required supplies of oxygen and hydrogen. This can be used for other processes either local to the biomass burning electrical power generating plant or more distantly, with the oxygen transported as the liquid. Alternatively oxygen may be transported as the gas, via a pipe network.

Excess oxygen may be employed, if desired, to burn the carbon based fuel produced in the plant, if it is desired to use the fuel as local source of heat and/or electrical power. Where this use is made of the carbon based fuel then the flue gases from combustion of the carbon based fuel may also be captured and optionally reused, as described herein with respect to the original flue gases produced from burning the biomass.

The electricity required for electrolysis of water may be electricity generated by the biomass fuelled electrical power generating plant or from any another source. A convenient other source of electricity for electrolysis of water may be from renewable energy sources such as wind, wave, tidal or solar power. The oxygen and hydrogen produced from electrolysis when electricity is available from such sources can be readily stored for future use in carbon based fuel production. Thus the energy system of the invention may further include connection to another renewable energy source of electricity in addition to the biomass fuelled electrical power generating plant. This can provide additional electrical power above that produced by the biomass fuelled electrical power generating plant, as required to run the processes of the system. Another biomass fuelled generator or generators may also be used to provide additional power.

The energy system described above has a number of advantages. The biomass fuelled electrical power generating plant provides renewable electrical power. Making use of carbon capture allows the flue gases to be utilised in producing carbon based fuels such as methane or methanol which can be stored and distributed for conventional uses such as industrial processes, and domestic heating and cooking. Methane or other carbon based fuels produced and used in this way do not add to global carbon dioxide as long as the biomass consumed is replaced by new growth of biomass. Thus the system can provide a carbon neutral source of electricity and/or carbon based fuel.

The system is flexible in that the electrical power generated can be used to supply customers and/or to power the carbon based fuel production requirements, including carbon capture and electrolysis and/or other processes as described below. Where power from sources such as wind, wave, tidal or solar is in short supply, the focus can be on electricity generation in the conventional manner to supply the grid. Where power from other sources is abundant the system can supply and even build stocks of methane, methanol, or other carbon based fuels.
The energy system described herein produces carbon based fuels derived from consumption of biomass. Thus according to another aspect the present invention provides a method of producing carbon based fuel using the energy system of the first aspect, the method comprising:
consuming biomass by conversion of the biomass to a charcoal and consuming the charcoal in a direct carbon fuel cell; capturing carbon containing flue gases from consuming the biomass; and producing carbon based fuel by reaction of the captured carbon containing flue gases and a source of hydrogen.

The biomass may comprise, consist of, or consist essentially of, wood.

Conveniently the biomass is burned in a biomass burning electrical power generating plant, which may use an oxy-fuel combustion process to facilitate the carbon capture. Alternatively pyrolysis to a charcoal and consumption of the charcoal in a direct carbon fuel cell plant may be employed. The Sabatier process as described herein may be employed to produce methane from carbon dioxide and hydrogen. Methanol may alternatively or additionally be produced as a carbon based fuel.

In addition or alternatively to a plant for the production of methane, the energy system of the invention may include process equipment to allow production of other products from the captured carbon. For example the system may include a plant for the production of methanol. Methanol provides a convenient fuel for vehicle engines, for example.

Methanol may be produced directly from captured carbon dioxide according to the following general equation:

CO₂ + 3H₂ → CH₃OH + H₂O

The reaction may be carried out at elevated temperature and pressure in the presence of a catalyst, such as a copper containing catalyst (e.g. Cu/ZnO), in the known manner. Methanol may also be produced by reaction of the methane produced. The process may be carried out conventionally via steam reforming of methane to synthesis gas (CO and H₂); the water gas shift reaction to adjust the ratio of CO to hydrogen (with removal of CO₂) followed by synthesis by reaction of hydrogen with CO to give the methanol. If this procedure is followed the CO₂ removed can of course be recycled to produce more methane.

Alternatively, the methanol may be made more directly from methane. For example by partial oxidation of methane (catalytic oxy-reforming of methane) and conversion of the resulting carbon monoxide and hydrogen to methanol, in accordance with the following two equations:

CH₄ + 0.5O₂ → CO + 2H₂

CO + 2H₂ → CH₃OH

(Reference: Energy-efficient syngas production through catalytic oxy-methane reforming reactions; Choudhary T.V., Choudhary V.R. ; Angew. Chem. Int. Ed. Eng. 2008;47(10):1828-47.)

As a yet further alternative, if the burning of the biomass is controlled to produce carbon monoxide in significant quantities, then the reaction of the carbon monoxide with hydrogen can be used to produce methanol.

CO + 2H₂ → CH₃OH

The reaction may be carried out at elevated temperature and pressure in the presence of a catalyst, such as a copper containing catalyst, such as a mixture of copper, zinc oxide and alumina; in the known manner.

Where methanol is produced it may be further processed in the known manner to provide synthetic hydrocarbon based fuels such as gasoline, diesel or jet fuel.

### Brief Description of the Drawings

Figure 1 shows a schematic drawing illustrating aspects of the system of the invention; and
Figure 2 shows a schematic drawing illustrating a direct carbon fuel cell arrangement.

### Detailed Description of the Drawings and of Some Examples Illustrating Embodiments of the Invention

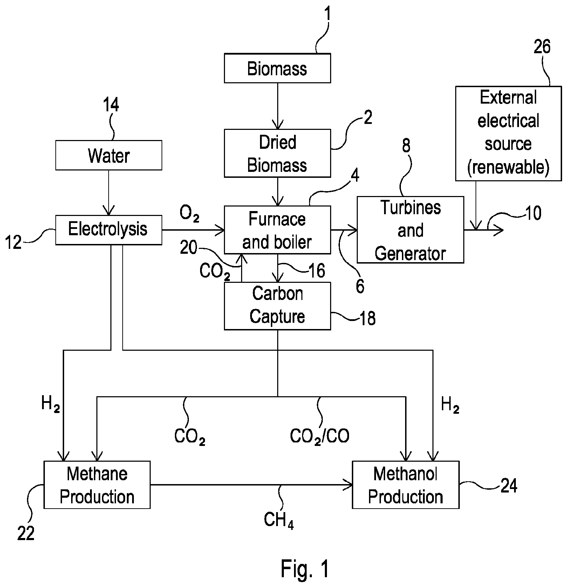

In schematic figure 1 the operation of an energy system including the production of methane and/or the production of methanol is shown. Only one of the methane plant or the methanol plant may be provided as both convert the biomass, after combustion to flue gas, to carbon based fuel. For flexibility the system may include plant suitable for production of both methane and methanol.

Biomass 1, such as wood, typically in the form of pellets, is dried 2 before being burned in the furnace and boiler arrangement 4 of a biomass burning electricity generator. The steam 6 produced from the boiler is used to drive a turbine and electrical generator arrangement 8 to give an electricity output 10.

The furnace is fed with oxygen from a water electrolysis plant 12, as the oxidant for the biomass, in the example shown. Alternatively or additionally air, or oxygen enriched air, or oxygen from another source such as an air separator may be supplied to support combustion. The water supply 14 for electrolysis may include any one of water driven off from drying the biomass, and water obtained from cooling the flue gases 16 from the furnace as they are processed in the carbon capture unit 18. Heat obtained from cooling processes such as when condensing the flue gases may be employed to dry the biomass.

Carbon capture unit 18 captures carbon dioxide and/or carbon monoxide from the flue gases. These captured gasses can be stored and/or used directly for the other processes described below.

In some cases carbon dioxide produced may be sent on for sequestration (permanent storage), for example in a geological formation, if removal of carbon from circulation in the atmosphere is desired.

As required a portion of the flue gases 16 or of gases such as carbon dioxide captured in capture unit 18 may be returned 20 to the furnace to dilute the oxygen content therein, moderating the combustion conditions. It may be convenient to mix these returning gases with the input of fresh oxygen, or other combustion gas mixture, before providing to the furnace.

Carbon dioxide is delivered from the carbon capture unit 18, typically via a storage facility (not shown), to the methane production unit 22, where reaction with hydrogen produces methane which is stored and/or sent on for use as required. The hydrogen required may be produced by the electrolysis unit 12 or obtained from elsewhere.

Also shown in figure 1 is a methanol production unit 24 which may be fed with carbon dioxide from carbon capture unit 18, carbon monoxide from carbon capture unit 18 (if some incomplete combustion is employed in the furnace), or methane from methane production unit 18, depending on the process or processes employed. Hydrogen required for methanol production may be obtained from electrolysis unit 12. Methanol can be stored and sent for use as a fuel for vehicles, or converted to synthetic hydrocarbon fuels for vehicles, for example.

The system shown in figure 1 can make use of the electricity generated 10 to supply power for the electrolysis unit 12, carbon capture unit 18, methane production unit 22 and any other requirements, such as methanol production 24. Surplus electricity may be supplied to other users (e.g. the electricity national grid). If desired the methanol produced may be further processed to synthetic hydrocarbon based fuels such as gasoline, diesel or jet fuel.

if the electricity 10 provided is insufficient to power the demand for methane and/or methanol, then an external source may optionally be employed. A renewable external source 26 is shown in figure 1, providing electricity from, for example, wind, wave, solar or tidal sources. Other biomass burning generators may be used as source 26.

If electricity is most wanted, rather than methane (or methanol, if a methanol production unit is provided) then the system used in figure 1 may be operated to maximise the electricity output 10, even at the expense of reduced or even ceased methane and/or methanol production. When electricity is sufficiently available from elsewhere the system may operate to maximise methane and/or methanol production.

Thus the system described is flexible in outputs and can help to smooth the variation in power supply from sources that are intrinsically variable in output, such as wind, solar, wave and tidal.

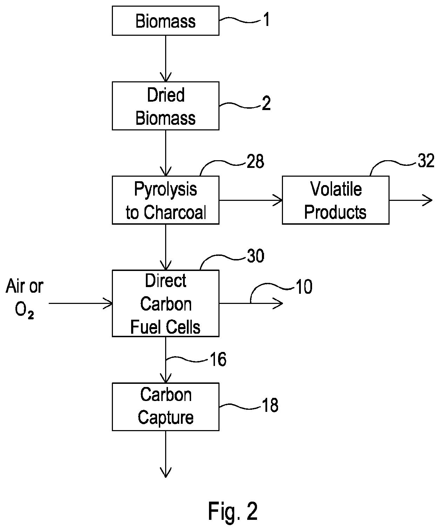

As an alternative to the biomass burning electricity generator arrangement shown in figure 1, a fuel cell based system may be employed. As depicted in the schematic figure 2 the biomass 1 is (optionally) dried 2, before being pyrolised to a charcoal 28 in the known manner. The charcoal (typically after a grinding process to produce a powder) is then used to generate electricity 10 in direct carbon fuel cells 30 which are also supplied with air or oxygen such as from electrolysis as depicted in the arrangements of figure 1.

The flue gases 16 from the fuel cells 30 are then subject to carbon capture in unit 18 for subsequent processing using any of the options as depicted in figure 1. Volatile products (gases and liquids) 32 from pyrolysis 28; such as methane, carbon monoxide, hydrogen and organic liquids; can be separated and utilised as appropriate. For example methane or methanol from or produced from the volatile pyrolysis products may be added to the stock prepared in a methane production unit 22 or methanol production unit 24 (see figure 1).

## Claims

1. An energy system comprising:
a biomass fuelled electrical power generating plant;
a carbon capture system for capturing carbon containing flue gases from consuming the biomass as fuel; and
a plant for producing carbon based fuel by reaction of the captured carbon containing flue gases and a source of hydrogen;
wherein the electrical power generating plant comprises:
a) a biomass pyrolising plant for pyrolising biomass to a charcoal; and
b) a direct carbon fuel cell plant for generating electricity from the charcoal.

2. The energy system of claim 1 further comprising connection to a source of electricity derived from at least one of wind, wave, solar, tidal and biomass burning.

3. Use of the energy system of claim 1 or 2 wherein the biomass fuelled electrical power generating plant burns biomass in air, optionally wherein at least one of:
the biomass is burned in an oxy-fuel combustion plant that optionally recycles a portion of flue gases to a boiler furnace; and
the burning of the biomass is controlled to produce carbon monoxide and the reaction of the carbon monoxide with hydrogen is used to produce methanol.

4. Use of the energy system of claims 1 or 2 wherein at least one of:
the plant for producing carbon based fuel is constructed and configured to produce either methanol or methane or both methanol and methane;
the system further comprises an electrolysis plant for the production of oxygen and hydrogen; and
capture of carbon containing flue gases includes compression to the liquid phase of carbon dioxide or carbon monoxide present in the flue gases.

5. Use according to claim 4 wherein said methane is produced from carbon dioxide captured from the flue gases, optionally by means of the Sabatier process.

6. Use according to any one of claims 3 to 5 wherein methanol is produced directly from carbon dioxide captured from the flue gases, optionally directly captured carbon dioxide according to the following general equation:
CO₂ + 3H₂ → CH₃OH + H₂O.

7. Use according to any one of claims 4 to 5 wherein methanol is produced from methane, optionally
by steam reforming of methane to synthesis gas; the water gas shift reaction to adjust the ratio of CO to hydrogen, with removal of CO₂; and then synthesis of methanol by reaction of hydrogen with CO; or optionally
by catalytic oxy-reforming of methane and conversion of the resulting carbon monoxide and hydrogen to methanol, in accordance with the following two equations:
CH₄ + O.5O₂ → CO + 2H₂
CO + 2H₂ → CH₃OH.

8. A method of producing carbon based fuel, using the energy system of claim 1, the method comprising:
consuming biomass by conversion of the biomass to a charcoal and consuming the charcoal in a direct carbon fuel cell ;
capturing carbon containing flue gases from consuming the biomass; and
producing carbon based fuel by reaction of the captured carbon containing flue
gases and a source of hydrogen.

9. The method of claim 8 wherein:
the biomass consumed comprises wood;
the carbon based fuel is selected from the group consisting of methanol, methane, and both methanol and methane; and/or
capturing carbon containing flue gases includes compression to the liquid phase of carbon dioxide or carbon monoxide present in the flue gases.

10. The method of claim 9, wherein methane is produced from carbon dioxide captured from the flue gases, optionally, wherein methane is produced from carbon dioxide captured from the flue gases by means of the Sabatier process.

11. The method of claim 9 wherein methanol is produced directly from carbon dioxide captured from the flue gases.

12. The method of claim 11 wherein methanol is produced directly from carbon dioxide and the said methanol is produced directly from captured carbon dioxide according to the following general equation:
CO₂ + 3H₂ → CH₃OH. + H₂O.

13. The method of claim 9, wherein the methanol is produced from methane, and
wherein the methanol is produced from methane by steam reforming of methane to synthesis gas; the water gas shift reaction to adjust the ratio of CO to hydrogen, with removal of CO₂; and then synthesis of methanol by reaction of hydrogen with CO.

14. The method of claim 9 wherein:
The methanol is produced from methane;
the methanol is produced from methane by catalytic oxy-reforming of methane and conversion of the resulting carbon monoxide and hydrogen to methanol, in accordance with the following two equations:
CH₄ + O.5O₂ → CO + 2H₂
CO + 2H₂ → CH₃OH.

15. The method of any one of claims 8 and 9 wherein the biomass is burned and burning of the biomass is controlled to produce carbon monoxide and the reaction of the carbon monoxide with hydrogen is used to produce methanol.

## Patentansprüche

1. Energiesystem, Folgendes beinhaltend:
eine biomassegefeuerte Stromerzeugungsanlage;
ein Kohlenstoff-Fangsystem zum Fangen von kohlenstoffhaltigen Abgasen aus dem Verbrauch von Biomasse als Brennstoff; und
eine Anlage zum Erzeugen von kohlenstoffbasiertem Brennstoff durch Reaktion der gefangenen kohlenstoffhaltigen Abgase mit einer Wasserstoffquelle;
wobei die Stromerzeugungsanlage Folgendes beinhaltet:
a) eine Biomasse-Pyrolyseanlage zum Pyrolysieren von Biomasse zu Holzkohle; und
b) eine Direkt-Kohlenstoff-Brennstoffzellenanlage zum Erzeugen von Strom aus der Holzkohle.

2. Energiesystem nach Anspruch 1, zudem beinhaltend eine Verbindung mit einer Elektrizitätsquelle , welche von mindestens einem von Wind, Wellen, Sonne, Gezeiten und Biomasseverbrennung stammt.

3. Gebrauch des Energiesystems nach Anspruch 1 oder 2, bei welchem die biomassegefeuerte Stromerzeugungsanlage Biomasse in Luft verbrennt, wobei optionsweise mindestens eines der folgenden Merkmale zutrifft:
die Biomasse wird in einer Sauerstoff-Brennstoff Verbrennungsanlage verbrannt, welche optionsweise einen Anteil von Abgasen an einen Heizkesselofen umwälzt; und
das Verbrennen der Biomasse wird gesteuert, um Kohlenmonoxid zu erzeugen und die Reaktion des Kohlenmonoxids mit Wasserstoff wird zum Erzeugen von Methanol verwendet.

4. Gebrauch des Energiesystems nach Anspruch 1 oder 2, bei welchem mindestens eines der folgenden Merkmale zutrifft:
die Anlage zum Erzeugen von kohlenstoffbasiertem Brennstoff ist konstruiert und konfiguriert, um entweder Methanol oder Methan oder um Methanol und Methan gleichermaßen zu erzeugen;
das System beinhaltet zusätzlich eine Elektrolyseanlage zum Erzeugen von Sauerstoff und Wasserstoff; und
das Fangen von kohlenstoffhaltigen Abgasen umfasst eine Kompression von in den Abgasen vorliegendem Kohlendioxid oder Kohlenmonoxid zur flüssigen Phase.

5. Gebrauch nach Anspruch 4, bei welchem das Methan aus Kohlendioxid erzeugt wird, welches aus den Abgasen gefangen wird, optionsweise mithilfe des Sabatier-Verfahrens.

6. Gebrauch nach einem der Ansprüche 3 bis 5, bei welchem Methanol direkt aus aus den Abgasen gefangenem Kohlendioxid erzeugt wird, optionsweise aus direkt gefangenem Kohlendioxid gemäß der folgenden allgemeinen Gleichung:
CO₂ + 3H₂ →CH₃OH + H₂O.

7. Gebrauch nach einem der Ansprüche 4 bis 5, bei welchem Methanol aus Methan erzeugt wird, optionsweise:
durch Methandampfreformierung zu Synthesegas; Wassergas-Shift-Reaktion zum Anpassen des Verhältnisses von CO zu Wasserstoff, unter Entfernung von CO₂; und anschließend Synthese von Methanol durch Reaktion von Wasserstoff mit CO; oder optionsweise
durch katalytische Sauerstoff-Reformierung von Methan und Umwandlung des resultierenden Kohlenmonoxids und Wasserstoffs in Methanol, gemäß den beiden folgenden Gleichungen:
CH₄ + 0.5O₂ → CO + 2H₂
CO + 2H₂ → CH₃OH.

8. Verfahren zum Erzeugen von kohlenstoffbasiertem Brennstoff unter Verwendung des Energiesystems nach Anspruch 1, wobei das Verfahren Folgendes beinhaltet:
Verbrauchen von Biomasse durch Umwandeln der Biomasse in eine Holzkohle und Verbrauchen der Holzkohle in einer Direkt-Kohlenstoff-Brennstoffzelle;
Fangen von kohlenstoffhaltigen Abgasen aus dem Verbrauch von Biomasse; und
Erzeugen von kohlenstoffbasiertem Brennstoff durch Reaktion der gefangenen kohlenstoffhaltigen Abgase mit einer Wasserstoffquelle.

9. Verfahren nach Anspruch 8, wobei:
die verbrauchte Biomasse Holz beinhaltet;
der kohlenstoffbasierte Brennstoff aus der Gruppe gewählt ist, bestehend aus Methanol, Methan und aus Methanol und Methan gleichermaßen; und/oder
das Fangen von kohlenstoffhaltigen Abgasen eine Kompression von in den Abgasen vorliegendem Kohlendioxid oder Kohlenmonoxid zur flüssigen Phase umfasst.

10. Verfahren nach Anspruch 9, bei welchem Methan aus Kohlendioxid erzeugt wird, welches aus den Abgasen gefangen wird, wobei optionsweise Methan aus Kohlendioxid erzeugt wird, welches aus den Abgasen mithilfe des Sabatier-Verfahrens gefangen wird.

11. Verfahren nach Anspruch 9, bei welchem Methanol direkt aus aus den Abgasen gefangenem Kohlendioxid erzeugt wird.

12. Verfahren nach Anspruch 11, bei welchem Methanol direkt aus Kohlendioxid erzeugt wird und das Methanol direkt aus gefangenem Kohlendioxid nach der folgenden allgemeinen Gleichung erzeugt wird:
CO₂ + 3H₂ → CH₃OH + H₂O.

13. Verfahren nach Anspruch 9, bei welchem Methanol aus Methan erzeugt wird, und
wobei das Methanol aus Methan erzeugt wird durch Methandampfreformierung zu Synthesegas; Wassergas-Shift-Reaktion zum Anpassen des Verhältnisses von CO zu Wasserstoff, unter Entfernung von CO₂; und anschließend Synthese von Methanol durch Reaktion von Wasserstoff mit CO.

14. Verfahren nach Anspruch 9, wobei:
das Methanol aus Methan erzeugt wird;
das Methanol aus Methan durch katalytische Sauerstoff-Reformierung von Methan und Umwandlung des resultierenden Kohlenmonoxids und Wasserstoffs in Methanol gemäß den beiden folgenden Gleichungen erzeugt wird:
CH₄ + 0.5O₂ → CO + 2H₂
CO + 2H₂ → CH₃OH.

15. Verfahren nach einem der Ansprüche 8 und 9, bei welchem die Biomasse verbrannt wird und Verbrennen der Biomasse gesteuert wird, um Kohlenmonoxid zu erzeugen und die Reaktion von Kohlenmonoxid mit Wasserstoff zum Erzeugen von Methanol verwendet wird.

## Revendications

1. Système de production d'énergie comprenant :
une centrale électrique dont le combustible est une biomasse ;
un système de capture de carbone pour capturer les gaz effluents contenant du carbone qui sont issus de la consommation de la biomasse en tant que combustible ; et
une installation pour produire du combustible à base de carbone par réaction des gaz effluents contenant du carbone capturés et d'une source d'hydrogène ;
dans lequel la centrale électrique comprend :
a) une installation de pyrolyse de biomasse pour pyrolyser la biomasse selon un charbon de bois ; et
b) une centrale à pile à combustible au carbone directe pour générer de l'électricité à partir du charbon de bois.

2. Système de production d'énergie selon la revendication 1, comprenant en outre une connexion sur une source d'électricité qui est dérivée d'au moins l'un parmi le vent, les vagues, le soleil, la marée et la combustion d'une biomasse.

3. Utilisation du système de production d'énergie selon la revendication 1 ou 2, dans laquelle la centrale électrique dont le combustible est une biomasse réalise la combustion de la biomasse dans l'air, en option dans laquelle au moins l'une des assertions qui suivent est satisfaite :
la biomasse subit une combustion dans une centrale de combustion oxygaz qui, en option, recycle une partie des gaz effluents sur un four de chaudière ; et
la combustion de la biomasse est commandée pour produire du monoxyde de carbone, et la réaction du monoxyde de carbone avec de l'hydrogène est utilisée pour produire du méthanol.

4. Utilisation du système de production d'énergie selon la revendication 1 ou 2, dans laquelle au moins l'une des assertions qui suivent est satisfaite :
l'installation pour produire du combustible à base de carbone est construite et configurée pour produire soit du méthanol, soit du méthane, soit à la fois du méthanol et du méthane ;
le système comprend en outre une installation d'électrolyse pour la production d'oxygène et d'hydrogène ; et
la capture de gaz effluents contenant du carbone inclut une compression en la phase liquide du dioxyde de carbone ou du monoxyde de carbone qui est présent dans les gaz effluents.

5. Utilisation selon la revendication 4, dans laquelle ledit méthane est produit à partir de dioxyde de carbone qui est capturé à partir des gaz effluents, en option au moyen du processus de Sabatier.

6. Utilisation selon l'une quelconque des revendications 3 à 5, dans laquelle le méthanol est produit directement à partir de dioxyde de carbone qui est capturé à partir des gaz effluents, en option à partir de dioxyde de carbone qui est capturé directement conformément à l'équation générale qui suit :
CO₂ + 3H₂ → CH₃OH + H₂O .

7. Utilisation selon l'une quelconque des revendications 4 à 5, dans laquelle le méthanol est produit à partir de méthane, en option :
par reformage à la vapeur de méthane en du gaz de synthèse ; par la réaction de conversion à la vapeur d'eau pour régler le rapport CO sur hydrogène, avec suppression du CO₂ ; puis par la synthèse du méthanol par réaction de l'hydrogène avec le CO ; ou en option
par oxy-reformage catalytique de méthane et par conversion du monoxyde de carbone et de l'hydrogène résultants en du méthanol, conformément aux deux équations qui suivent :
CH₄ + 0.5O₂ → CO + 2H₂
CO + 2H₂ → CH₃OH.

8. Procédé de production de combustible à base de carbone, en utilisant le système de production d'énergie selon la revendication 1, le procédé comprenant :
la consommation d'une biomasse par conversion de la biomasse en un charbon de bois et la consommation du charbon de bois dans une pile à combustible au carbone directe ;
la capture des gaz effluents contenant du carbone qui sont issus de la consommation de la biomasse ; et
la production de combustible à base de carbone par réaction des gaz effluents contenant du carbone capturés et d'une source d'hydrogène.

9. Procédé selon la revendication 8, dans lequel :
la biomasse consommée comprend du bois ;
le combustible à base de carbone est sélectionné parmi le groupe qui est constitué par le méthanol, le méthane et à la fois le méthanol et le méthane ; et/ou
la capture des gaz effluents contenant du carbone inclut une compression en la phase liquide du dioxyde de carbone ou du monoxyde de carbone qui est présent dans les gaz effluents.

10. Procédé selon la revendication 9, dans lequel le méthane est produit à partir du dioxyde de carbone qui est capturé à partir des gaz effluents, en option dans lequel le méthane est produit à partir du dioxyde de carbone qui est capturé à partir des gaz effluents au moyen du processus de Sabatier.

11. Procédé selon la revendication 9, dans lequel le méthanol est produit directement à partir de dioxyde de carbone qui est capturé à partir des gaz effluents.

12. Procédé selon la revendication 11, dans lequel le méthanol est produit directement à partir de dioxyde de carbone et ledit méthanol est produit directement à partir de dioxyde de carbone capturé conformément à l'équation générale qui suit :
CO₂ + 3H₂ CH₃OH. + H₂O.

13. Procédé selon la revendication 9, dans lequel le méthanol est produit à partir de méthane ; et
dans lequel le méthanol est produit à partir de méthane par reformage à la vapeur de méthane en du gaz de synthèse ; par la réaction de conversion à la vapeur d'eau pour régler le rapport CO sur hydrogène, avec suppression du CO₂ ; puis par la synthèse du méthanol par réaction de l'hydrogène avec le CO.

14. Procédé selon la revendication 9, dans lequel :
le méthanol est produit à partir de méthane ;
le méthanol est produit à partir de méthane par oxy-reformage catalytique de méthane et par conversion du monoxyde de carbone et de l'hydrogène résultants en du méthanol, conformément aux deux équations qui suivent :
CH₄ + 0.5O₂→ CO + 2H₂
CO + 2H₂ → CH₃OH.

15. Procédé selon l'une quelconque des revendications 8 et 9, dans lequel la biomasse subit une combustion et la combustion de la biomasse est commandée pour produire du monoxyde de carbone, et la réaction du monoxyde de carbone avec de l'hydrogène est utilisée pour produire du méthanol.
